Europäisches Patentamt

European Patent Office

Office européen des brevets

(11) Veröffentlichungsnummer: **0 192 222**
**B1**

(12) **EUROPÄISCHE PATENTSCHRIFT**

(45) Veröffentlichungstag der Patentschrift: **29.08.90**

(51) Int. Cl.$^5$: **A 61 K 33/00** // A23K1/175, A23L1/05

(21) Anmeldenummer: **86102026.1**

(22) Anmeldetag: **18.02.86**

(54) **Mittel zur Verhinderung, Verminderung oder Verzögerung der Eiweissfäulnis im Verdauungstrakt von Mensch oder Tier und Verfahren zur Herstellung eines solchen Mittels.**

(30) Priorität: **19.02.85 DE 3505628**

(43) Veröffentlichungstag der Anmeldung:
**27.08.86 Patentblatt 86/35**

(45) Bekanntmachung des Hinweises auf die Patenterteilung:
**29.08.90 Patentblatt 90/35**

(84) Benannte Vertragsstaaten:
**AT BE CH FR IT LI NL**

(56) Entgegenhaltungen:
EP-A-0 024 891      DE-A-2 924 416
EP-A-0 147 932      DE-A-3 230 129
EP-A-0 150 391      DE-A-3 246 278
DE-A-2 833 727      FR-A-2 333 449

PATENT ABSTRACTS OF JAPAN, Band 1, Nr. 64 (C-77), 22. Juni 1977, Seite 843 C 77; & JP-A-52 24 876 (PEARLSTONE KOGYO K.K.) 24-02-1977

Die Akte enthält technische Angaben, die nach dem Eingang der Anmeldung eingereicht wurden und die nicht in dieser Patentschrift enthalten sind.

(73) Patentinhaber: **Deutsche Perlite GmbH**
**Kipperstrasse 19**
**D-4600 Dortmund 1 (DE)**

(72) Erfinder: **Knauf, Albrecht, Dr.-Ing.**
**Kipperstrasse 19**
**D-4600 Dortmund 1 (DE)**

(74) Vertreter: **Becker, Thomas, Dr., Dipl.-Ing. et al**
**Patentanwälte Becker & Müller**
**Eisenhüttenstrasse 2**
**D-4030 Ratingen 1 (DE)**

(56) References cited:
COMMONWEALTH AGRICULTURAL BUREAU, Zusammenfassung Nr. 0N047-05577, Nutrition Abs. and Rev./Ser.B; F.G. PROUDFOOT et al.: "Effects of feeding glass particles of different sizes to broiler chickens", & Canadian Journal of Animal Science, Band 56, Nr. 1, 1976, Seiten 99-100

**Beschreibung**

Mittel zur Verhinderung, Verminderung oder Verzögerung der Eiweißfäulnis im Verdauungstrakt von Mensch oder Tier bestehen üblicherweise aus chemisch und biochemisch wirksamen Substanzen, die regelmäßig sowohl erwünschte als auch unerwünschte Wirkungen auf den Organismus ausüben. Insbesondere die unerwünschten Eigenschaften derartiger Mittel lassen es angezeigt erscheinen, mit dem Umgang dieser Mittel besonders zurückhaltend zu sein. Dies gilt insbesondere in Hinblick auf den Bereich der Arzneimittel, die auf die Verdauung einwirken oder auf die für die Ernährung von Mensch und Tier verwendeten Nahrungsmittel.

Nahrungsmittel bestehen im wesentlichen aus Proteinen, Kohlehydraten, Fetten und Wirkstoffen. Letzere setzen sich aus Vitaminen und Mineralstoffen zusammen. Bei den Proteinen unterschedet man zwischen pflanzlichen und tierischen. Im Gegensatz zum Menschen und den meisten Tieren können Pflanzen Proteine selbst erzeugen. Aus diesem Grunde müssen Proteine von Mensch und Tier mit der Nahrung aufgenommen werden, um körpereigenes Protein in Form von Fleisch produzieren beziehungsweise erhalten zu können.

Während sich Tiere hauptsächlich von Pflanzen ernähren, nimmt der Mensch sowohl pflanzliche als auch tierische Produkte mit der Nahrung auf. Der Gehalt an proteinen in den einzelnen Nährstoffträgern ist sehr unterschiedlich.

Hierzu einige Beispiele:
Fisch 40 — 50%
Fleisch 20 — 30%
Hühnerei 14%
Sojabohne 40%
· Mais 11%

Proteine bestehen aus Aminosäuren. Bisher sind 22 unterschiedliche Aminosäuren bekannt. Zum Aufbau körpereigener Proteine bei Mensch und Tier müssen alle Aminosäuren in der richtigen Menge dem Organismus zur Verfügung gestellt werden. Fehlt eine dieser Aminosäuren, so ist die Proteinsynthese nicht möglich. Bei den Aminosäuren unterscheidet man zwischen essentiellen und nichtessentiellen. Die essentiellen Aminosäuren unterscheiden sich von den nichtessentiellen dadurch, daß sie von Mensch und Tier in ausreichender Menge aufgenommen werden müssen, da sie vom Organismus nicht aus anderen Aminosäuren umgebaut werden können und in den Nährstoffträgern nur in begrenzter Menge vorhanden sind.

Die Energieversorgung von Mensch und Tier erfolgt im wesentlichen über die Aufnahme von Kohlehydraten oder Fetten. Es ist selbstverständlich, daß Lebewesen nur dann körpereigenes Protein bilden können, wenn sie ausreichend mit Energie und Wirkstoffen versorgt werden.

Diese Zusammenhänge sollen am Beispiel eines Schweins verdeutlicht werden, wobei dieses Beispiel auch stellvertretend für andere Tiere oder auch den Menschen gewählt wurde.

Ein Schwein mit einem Gewicht von ca. 100 kg Lebendmasse besteht zu ca. 16 kg aus Protein und ca. 23 kg aus Fett. Der Rest setzt sich hauptsächlich aus Wasser und Knochen zusammen. Zur Erzeugung von 16 kg körpereigenem Protein muß das Schwein etwa die doppelte Menge an pflanzlichen Rohproteinen aufnehmen. Diese Menge reicht jedoch nur aus, wenn alle essentiellen Aminosäuren darin ausreichend enthalten sind.

In der Mast eines Schweines von 20 bis 100 kg Lebendgewicht müssen dem Tier nach der herrschenden Lehre etwa folgende Mengen an essentiellen Aminosäuren zur Verfügung stehen:

Lysin 1,90 kg
Methionin und Cystin 1,25 kg
Tryptophan 0,38 kg
Threonin 1,00 kg

In der Praxis ist es jedoch in den seltensten Fällen möglich, ein Futter auf den richtigen Gehalt aller Aminosäuren abzustimmen.

Während in der Schweinemast von 20 bis 100 kg Lebendmasse ein Mindestgehalt an Rohprotein im gesamten Futter von 25 bis 27 kg gefordert wird, beträgt der tatsächliche Gehalt meist zwischen 40 und 50 kg. Demnach beträgt der Rohproteingehalt im Futter annähernd das Doppelte der geforderten Menge. Der Grund für den hohen Rohproteingehalt im Schweinefutter liegt darin, daß die Aminosäuren in den pflanzlichen Rohproteinen in einem anderen Verhältnis enthalten sind, als es für die Fütterung eines Tieres notwendig ist.

Der größte Teil der von den Tieren aufgenommenen Rohproteine wird im Verdauungstrakt mit Hilfe von Enzymen beziehungsweise Bakterien in die einzelnen Aminosäuren gespalten und in den Körperzellen zu körpereigenem Protein umgebaut. Der Überschuß an Aminosäuren wird über die Nieren mit dem Harn als Harnstoff ausgeschieden.

Demgegenüber wird der kleinere Teil der von den Tieren aufgenommenen Rohproteine jedoch nicht oder nur unvollständig abgebaut. Diese Rohproteine verbleiben im Verdauungstrakt und können von Bakterien, die in ausreichender Menge bei allen Tieren vorhanden sind, zersetzt werden. Diese bakterielle Zersetzung von Rohproteinen wird als Eiweißfäulnis bezeichnet. Bei der Eiweißfäulnis entstehen unter anderem Fäulnisprodukte, die zum Teil sehr giftig sind und vom Verdauungstrakt in die Blutbahn gelangen können. Diese giftigen Vebindungen werden von der Leber abgebaut und ebenfalls von den Nieren als Harnstoff ausgeschieden. Der Abbau der giftigen Verbindungen wird jedoch dann erschwert, wenn dem Menschen oder dem Tier zeitweise oder ständig eine zu große Menge an Rohproteinen zugeführt wird. Die giftigen Verbindungen reichern sich im Körper an und belasten den gesamten Organismus. Die Gesundheit kann damit stark beeinträchtigt werden. Diese am Beispiel eines Schweines dargestellten Verhältnisse lassen sich im wesentlichen auch auf andere Tiere wie zum Beispiel Rinder, Schafe, Geflügel, Kaninchen, Fisch usw. und auch auf den Menschen übertragen. Dabei versteht sich, daß sich im einzelnen Abweichungen ergeben können.

Bedingt durch eine zu proteinreiche Ernährung stellt sich im Organismus eine latente Vergiftung ein. Hierdurch können beispielsweise beim Menschen Konditionsmängel, Konstitutionsmängel und als Folge davon ein eingeschränktes Leistungsvermögen festgestellt werden. Derartige Erscheinungen sind besonders in Zeiten wirtschaftlichen Überflusses festzustellen.

Aus der Literatur ist bekannt, daß in der Nahrung von Mensch und Tier auch Ballaststoffe enthalten sein müssen. Ballaststoffe sind schwerbeziehungsweise unverdauliche Nahrungsbestandteile. Hierzu gehören: Cellulose, Hemicellulose, Pektine, Lignin usw. Ein Teil der Ballaststoffe besteht aus Rohfaser. Ballaststoffe regen die Abgabe der Verdauungssäfte im Verdauungstrakt an, sie fördern die Darmperistaltik. Durch Ballaststoffe wird die Verweilzeit des Nahrungsmittels im Darm vermindert, die Darmflora günstig beeinflußt und schädliche beziehungsweise unerwünschte Stoffe werden adsorbiert.

Ein Mangel an Ballaststoffen beziehungsweise Rohfaser kann zur Erkrankung oder sogar zum Tod von Mensch oder Tier fuhren. Versuche bei Mastschweinen mit rohfaserarmen Futtermitteln wie Molke oder Magermich zeigen, daß die Sterblichkeitsrate besonders hoch ist.

In der Schweinezucht werden die Sauen mit einem wesentlich höheren Anteil an Rohfaser als die Mastschweine gefüttert. Durch den höheren Rohfasergehalt, der sich im Verdauungstrakt sehr positiv auswirkt, erzielt man gesunde Muttertiere, die dann wiederum in der Lage sind, gesunde Ferkel zu erzeugen.

Der Rohfasergehalt im Schweinemastfutter ist jedoch begrenzt, da die Vorteile, die sich durch die Rohfaser ergeben, durch den Nachteil geringerer Mastleistung aufgehoben werden. Ein Teil der Rohfaser wird im Darm des Schweines bakteriell zersetzt. Für diese Zersetzung wird Energie benötigt, die dem Schwein zum Teil entzogen wird und die andernfalls für das Wachstum zur Verfügung stehen würde.

Da die Sauen in der Zucht keine Wachstumsleistung erbringen müssen, kann bei der Sauenfütterung der Rohfasergehalt deutlich erhöht werden.

Wissenschaftliche Untersuchungen belegen, daß mit steigendem Rohfasergehalt in der Fütterung von Schweinen ein steigender Anteil an giftig wirkenden Stoffen aus dem Darm absorbiert wird.

Aufgabe der vorliegenden Erfindung ist es, eine Möglichkeit aufzuzeigen, wie unter Vermeidung chemisch oder biochemisch wirksamer Substanzen dennoch durch geeignete Mittel die Gesundheit von Menschen und Tieren, letzterer insbesondere in der Mast, erhalten oder verbessert werden kann.

Diese Aufgabe wird erfindungsgemäß durch die Verwendung speziell aufbereiteter, im Verdauungstrakt nicht abbaubarer Gläser gemäß Anspruch 1 als Rohfaserersatz in Nahrungsmitteln zur Herstellung eines Arzneimittels gelöst, das der Behandlung (Verhinderung, Verminderung oder Verzögerung) der Eiweißfäulnis im Verdauungstrakt dient ausgenommen Schweinefutter mit einem Gehalt an geblähtem Perlite.

Die Wirkung beim Einsatz von natürlichen oder künstlichen Gläsern in der menschlichen oder tierischen Ernährung ist mit der Wirkung von Rohfaser zu vergleichen. Während Gläser die Eiweißfäulnis im Verdauungstrakt verhindern, vermindern oder verzögern, bewirkt die Rohfaser, daß giftig wirkende Stoffe an sie gebunden und mit dem Kot ausgeschieden werden.

Im Gegensatz zur Rohfser die aus organischen Substanzen besteht, sind die im Anspruch näher bezeichneten Gläser im Verdauungstrakt nicht abbaubar. Die geschilderten Nachteile, die die Rohfaser hat, entfallen deshalb.

Aus der DE-A-29 24 416 ist ein übliches Schweinefutter bekannt, dem zusätzlich Perlitekörner zugesetzt werden, die dabei die Funktion eines Füllstoffes haben. Während die feinkörnigen Teile des Perlits die unterschiedlich rauhen und zerklüfteten Oberflächen der einzelnen Futterkomponenten füllen sollen, verfüllen die feinen Mehlanteile der verschiedenen Futterkomponenten die offenen Poren des Perlites. Durch den Perlitezusatz wird eine ad libitum-Fütterung ermöglicht. Hinweise auf einen Ersatz von Rohfaser durch Perlite zur Herstellung eines Arzneimittels gibt die DE-A-29 24 416 nicht.

Aus den EP-A-24 B91 und EP-A-147 932 sind wasserlösliche Phosphatgläser bekannt, die die Aufgabe haben, Wirksubstanzen in den Organismus einzubringen und dann im Organismus durch Zersetzung die Wirkstoffe freizugeben. Auf diese Weise werden wiederum chemisch beziehungsweise biochemisch wirksame Substanzen in den Organismus eingeführt mit den dadurch bedingten, eingangs genannten Nachteilen.

In der DE-A-32 30 129 wird für die sogenannte restriktive oder rationierte Fütterung von Schweinen vorgeschlagen, Perlite in einer Menge von 10 bis 40 Vol.-%, bezogen auf die Gesamtfuttermenge, beizusetzen, um dem Schwein einerseits nach Maßgabe seiner individuellen physiologischen Bedürfnisse ein ständiges Sattfressen zu ermöglichen, infolge der Beimischung der Inertstoffe jedoch nur ein beschränktes Nährstoffangebot bereitzustellen. Auch hier liegt der Sinn und Zweck des Perlitezusatzes ausschließlich auf futtertechnischem Gebiet. Dies gilt auch für die aus Commonwealth Agricultural Bureau Abstr. Nr. ONO47-05577 beziehungsweise Patent abstract of Japan, Bd. I, Nr. 64 (C-77) bekannten Futtermittel für Hühner.

Die gesundheitsfördernde Wirkung konnte unter anderem bei Kälbern nachgewiesen werden, die mit einem Milchaustauscher gefüttert wurden, der rohfaserfrei war und der mit Glasmehl mit einer Körnung bis ca. 100 µm und einem

Anteil von 3% der Trockenmasse angemischt wurde. Hierbei zeigten sich überraschend gute Ergebnisse. Die Sterblichkeit, die vorher zwischen 5 und 10% lag, konnte fast völlig beseitigt werden. Das Fell der Tiere war glatter und glänzender und die Kälber hatten ein besseres Wachstum.

Desweiteren konnte nachgewiesen werden, daß der Abbau der im Kot und Urin befindlichen restlichen Rohproteine durch die Fütterung der Schweine mit geblähtem Perlit auch bei längerer Lagerung unter anaeroben Bedindungen in beachtlichem Maße verzögert wird.

Durch die Reduzierung der Gestankstoffe wird die Umweltbelastung sowohl für die in der Nähe eines Stalles lebenden Menschen als auch für die im Stall befindlichen Tiere stark herabgesetzt. Besonders auffällig ist, daß der in einem Schweinestall üblicherweise herrschende stechende Ammoniakgestank weitestgehend beseitigt wird.

Für die Verwendung der Gläser als Arzneimittel oder für die Ernährung ist es erforderlich, daß diese Gläser so aufbereitet werden, daß sie der Organismus verträgt. Dies kann insbesondere durch Mahlen beziehungsweise Feinmahlen, Aufschäumen oder Aufschäumen und Feinmahlen erreicht werden. Dabei können die Gläser vor dem Aufschäumen mit Zusätzen versehen werden. Außerdem können die Produkte durch Sieben oder Sichten von unerwünschten Fremdkörpern, wie sie in Naturstoffen regelmäßig vorkommen, befreit werden.

Die Aufbereitung eines natürlichen Glases soll am Beispiel von Perlite dargestellt werden. Das Material kann bei etwa 1.000°C zu einem sehr leichten, porösen Schüttgut aufgeschäumt werden. Durch das Aufschäumen vergrößert sich die Oberfläche des Materials um ein Vielfaches. Durch eine zusätzliche Mahlung erhält man eine weitere erwünschte Vergrößerung der Oberfläche des Materials. Eine ähnliche Aufbereitung kann auch mit künstlichen Gläsern vorgenommen werden. Dabei können Schäumungszusätze Verwendung finden.

Das Mittel kann als Arzneimittel in Tabletten oder Kapselform ausgebildet werden. Es kann ferner als Gel oder in einer Flüssigkeit, zum Beispiel Wasser, aufgeschwemmt sein.

Das Mittel soll im übrigen direkt als Rohfaser-Ersatzstoff in einem Nahrungsmittel eingesetzt werden beziehungsweise als Zusatz zu einem rohfaserhaltigen Nahrungsmittel Verwendung finden.

**Patentansprüche**

1. Verwendung mindestens eines, durch Mahlen beziehungsweise Feinmahlen, Aufschäumen oder Aufschäumen und Feinmahlen aufbereiteten, im Verdauungstrakt nicht abbaubaren natürlichen oder künstlich hergestellten Glases aus der Gruppe Bims, Lavagestein, Obsidian, Perlite und/ oder Pechstein als vollständiger oder teilweiser Rohfaserersatz in Nahrungsmitteln zur Herstellung eines Arzneimittels zur Behandlung (Verhinderung, Verminderung oder Verzögerung) der Eiweißfäulnis im Verdauungstrakt von Mensch oder Tier ausgenommen Schweinefutter mit einem Gehalt an geblähtem Perlite.

2. Verwendung nach Anspruch 1 mit der Maßgabe, daß das Glas ein durch thermische Vorbehandlung aufgeschäumtes Glas ist.

3. Verwendung nach einem der Ansprüche 1 bis 2 mit der Maßgabe, daß das Glas eine Korngröße bis zu 100 μm aufweist.

4. Verwendung nach einem der Ansprüche 1 bis 3 mit der Maßgabe, daß das Glas in Tablettenoder Kapselform konfektioniert ist.

5. Verwendung nach einem der Ansprüche 1 bis 3 mit der Maßgabe, daß das Glas in Mischung mit einer Flüssigkeit oder einem Gel konfektioniert ist.

6. Verwendung nach Anspruch 5 mit der Maßgabe, daß das Glas in Mischung mit Wasser konfektioniert ist.

7. Verfahren zur Herstellung eines Arzneimittels nach einem der Ansprüche 1 bis 6, dadurch gekennzeichnet, daß das gemahlene und/oder aufgeschäumte Glas vor der Konfektionierung von unerwünschten Fremdkörpern oder Beimengungen befreit wird.

**Revendications**

1. Utilisation d'au moins un verre, naturel ou artificiel, non dégradable dans le tube digestif, préparé par broyage ou broyage fin, moussage ou moussage et broyage fin, du groupe des pierres ponces, des laves, de l'obsidienne, de la perlite et/ou du pechstein, en remplacement total ou partiel des fibres crues, dans les aliments, pour la fabrication d'un médicament destiné à traiter (inhiber, réduire ou retarder) la décomposition protéinique, dans l'appareil digestif, chez l'homme ou l'animal, à l'exception des aliments pour porcs, contenant de la perlite expansée.

2. Utilisation selon la revendication 1, caractérisée en ce que le verre est un verre expansé en mousse, par traitement thermique préliminaire.

3. Utilisation selon la revendication 1 ou 2, caractérisée en ce que le verre a une granulométrie allant jusqu'à 100 μm.

4. Utilisation selon l'une quelconque des revendications 1 à 3, caractérisée en ce que le verre est conditionné sous forme de comprimés ou de capsules.

5. Utilisation selon l'une quelconque des revendications 1 à 3, caractérisée en ce que le verre est conditionné en mélange avec un liquide ou un gel.

6. Utilisation selon la revendication 5, caractérisée en ce que le verre est conditionné en mélange avec de l'eau.

7. Procédé de préparation d'un médicament selon l'une quelconque des revendications 1 à 6, caractérisé en ce qu'on élimine du verre broyé et/ ou expansé en mousse, les corps étrangers ou ajouts indésirables, avant le conditionnement.

## Claims

1. Use of at least one natural or artificially manufactured glass, which has been prepared by grinding, foaming or foaming and fine grinding and not being able to catabolize in the digestive system, from the group including pumice, lava, obsidian, perlite and/or pitchstone as a complete or partial substitute of crude fibers in foodstuffs for producing a pharmaceutic for treatment (prevention, reduction or retardation) of protein decay in the digestive system of man or animal, excluding pig food with a content of expanded perlite.

2. Use following claim 1 with the proviso that said glass is a glass which has been expanded by thermal pretreatment.

3. Use following one of claims 1 or 2 with the proviso that said glass has a particle size up to 100μm.

4. Use following one of claims 1 to 3 with the proviso that said glass is prepared in form of a tablet or a capsule.

5. Use following one of claims 1 to 3 with the proviso that said glass is prepared as a mixture with a fluid or a gel.

6. Use following claim 5 with the proviso that said glass is prepared as a mixture with water.

7. Process for manufacturing a pharmaceutic following one of claims 1 to 6, characterized in that said grinded and/or foamed glass is freed from undesired foreign substances or admixtures.